(19) Europäisches Patentamt
European Patent Office
Office européen des brevets

(11) Veröffentlichungsnummer: **0 173 019**
**A2**

# (12) EUROPÄISCHE PATENTANMELDUNG

(21) Anmeldenummer: **85107749.5**

(22) Anmeldetag: **22.06.85**

(51) Int. Cl.⁴: **C 07 C 51/09**, B 01 J 23/64

(30) Priorität: **21.08.84 DE 3430663**

(43) Veröffentlichungstag der Anmeldung: **05.03.86**
**Patentblatt 86/10**

(84) Benannte Vertragsstaaten: **BE DE FR GB IT NL**

(71) Anmelder: **HÜLS AKTIENGESELLSCHAFT, - RSP Patente / PB 15 - Postfach 13 20, D-4370 Marl 1 (DE)**

(72) Erfinder: **Hög, Hans-Ulrich, Dr., Bitterfelder Strasse 9 a, D-4370 Marl (DE)**
Erfinder: **Bub, Günther, Dr., Kampstrasse 94, D-4370 Marl (DE)**

(54) **Katalysatorsystem und Verfahren zur Herstellung von Carbonsäuren durch Umsetzung von Carbonsäureestern mit Ameisensäure.**

(57) Zur Herstellung von Carbonsäuren durch Umsetzung von Carbonsäureestern mit Ameisensäure setzt man ein Metallkatalysatorsystem aus Rhodium bzw. Rhodiumsalzen oder Rhodiumkomplexen, aus Halogen oder einer Halogenverbindung, aus einer Metallverbindung der VI. Nebengruppe, bevorzugt Chromcarbonyl, Chromacetat und/oder Chromhalogenide und/oder Liganden aus der Gruppe der organischen Stickstoff- oder Phosphorverbindungen als Promotor ein. Die Konzentration dieses Katalysatorsystems beträgt 0,05 bis 5, vorzugsweise 0,1 bis 2 mg-atom Rhodium pro mol eingesetzten Carbonsäureester. Die Umsetzung erfolgt bevorzugt in Gegenwart von Kohlenmonoxid in der Flüssigphase bei Temperaturen von 100 bis 300 °C und Kaltdrücken von 1 bis 250 bar.

EP 0 173 019 A2

Katalysatorsystem und Verfahren zur Herstellung von Carbonsäuren durch Umsetzung von Carbonsäureestern mit Ameisensäure

Die Erfindung betrifft ein Verfahren zur Herstellung von Carbonsäuren oder Gemischen von Carbonsäuren durch Umsetzung eines Carbonsäureesters mit Ameisensäure. Die Umsetzung erfolgt in weitgehend wasserfreiem Medium in Gegenwart eines Katalysatorsystems, bestehend aus einer Rhodiumkomponente, einer Jod- oder Bromkomponente als Promotor und einer weiteren Metallkomponente aus der VI. Nebengruppe oder einem Liganden aus der Gruppe der organischen Stickstoff- oder Phosphorverbindungen anstelle oder zusätzlich zu dieser zweiten Metallkomponente. Insbesondere befaßt sich die Erfindung mit der Herstellung von Essigsäure und Phenylessigsäure.

Eine Methode zur Durchführung von Reaktionen des Typs der Gleichung 1 beschreibt die EP-PS 045 637.

$$R^1COOR^2 + HCOOH \longrightarrow R^1COOH + R^2COOH \qquad (Gl. 1)$$

Sie besteht in der Umsetzung der Reaktionspartner in Gegenwart eines Katalysators aus einer löslichen Iridiumverbindung und einem Jodpromotor, ohne dazu Kohlenmonoxid in den Reaktor einzuführen. Aus dem in den Versuchsbeispielen angegebenen Druck, wie er sich bei der Reaktionstemperatur einstellt, und den Mengenbilanzen zwischen erhaltendem Produkt und Einsatz ist jedoch unschwer zu ersehen, daß ein Teil der Ameisensäure zersetzt wird; die Tendenz der Ameisensäure, sich gemäß den Gleichungen (2) und (3) thermisch, säure- oder metallkatalysiert zu zersetzen, ist wohlbekannt.

$$HCOOH \longrightarrow CO + H_2O \qquad (Gl.\ 2)$$
$$HCOOH \longrightarrow CO_2 + H_2 \qquad (Gl.\ 3)$$

So geht aus dem Beispiel 30 der EP-PS hervor, daß bei einer Umsetzung von Methylacetat mit Ameisensäure gemäß Gleichung 1 28 % mehr Ameisensäure als Methylacetat umgesetzt wurden. In der EP-PS wird die Ameisensäure als Reaktionspartner und Lösemittel zugleich bezeichnet, so daß ein zumindest kleiner Überschuß als günstig anzusehen ist.

Ausdrücklich werden Rhodiumkatalysatoren als unwirksam bezeichnet, wenn man dem Reaktionssystem kein Kohlenmonoxid zugibt (Beispiel 14 und 26 der EP-PS), und auch dann noch als deutlich weniger wirksam, wenn man die Umsetzung nach dem Aufpressen von Kohlenmonoxid durchführt (Beispiel 15 der EP-PS). Dabei beziehen sich die Beispiele 14 und 15 auf eine Variante der Reaktion gemäß Gleichung 4, die über ein Umesterungsgleichgewicht als der Gleichung 1 äquivalent angesehen werden kann.

$$HCOOCH_3 + RCOOH \longrightarrow CH_3COOH + RCOOH \qquad (Gl.\ 4).$$

Andererseits handelt es sich bei Iridium um ein besonders teures und nur in sehr beschränktem Maße verfügbares Material, so daß es für eine technische Anwendung vorteilhaft wäre, wenn es gelänge, die Reaktion mit einem wenigstens in etwa gleich wirksamen Rhodiumkatalysator durchzuführen.

Rhodiumkatalysierte Carbonylierungsreaktionen von Estern zu Carbonsäuren sind bekannt; beispielhaft soll die DE-PS 17 67 151 (= US-PS 3 689 533) genannt werden. Dabei wird jedoch Wasser in mindestens äquimolarer Menge zum Ester miteingesetzt; außerdem werden als Substrate Ester erst beginnend mit denen der Essigsäure,

nicht jedoch Ameisensäureester verwendet.
Ein in der DE-AS 19 66 695 (= US-PS 3 717 670) beschriebener Rhodium-Chrom-Trägerkatalysator unterliegt denselben Einschränkungen und wird nur bei Gasphasenreaktionen angewendet, wobei er deutlich weniger wirksam ist als die bekannten chromfreien Katalysatoren bei Umsetzungen in der Flüssigphase.

Ester der Ameisensäure lassen sich, außer nach der schon zitierten Variante gemäß Gleichung 4 aus der EP-PS 045 637, in Gegenwart von Edelmetallkatalysatoren und Kohlenmonoxid ohne eine Carbonsäure als unverzichtbarem Bestandteil des Reaktionsmediums umlagern; solche Verfahren sind durch die DE-PS 21 09 025 (= US-PS 3 798 267), US-PS 4 194 056, DE-OS 32 36 351, die unveröffentlichte DE-Anmeldung 33 33 317.3 (Rh + Halogenverbindung, gegebenenfalls weitere Komponenten), die JP-OS 56-22745 und die DE-PS 30 46 899 (Pd-, Ir- oder Ru- + Halogenverbindung sowie weitere Komponenten) bekannt. Die EP-Appl. 60 695 beschreibt die durch einen aus einer Rhodium- und einer Jodverbindung bestehenden Katalysator bewirkte Umsetzung gemäß Gleichung 5.

$$HCOOCH_3 + H_2O + CO \longrightarrow CH_3COOH + HCOOH \quad \text{(Gl. 5)}$$

Hieraus ergab sich die Aufgabe, ein einfaches und wirtschaftliches Verfahren zur Herstellung von Carbonsäuren zu finden.

Diese Aufgabe wird erfindungsgemäß entsprechend den Angaben der Patentansprüche gelöst.

Das erfindungsgemäße Katalysatorsystem auf Rhodiumbasis für die Reaktion nach Gleichung 1 ist neu.

Dem stehen auch nicht die Möglichkeiten entgegen, daß Ameisensäure durch Zersetzung zu Kohlenmonoxid und Wasser eine Verseifung des Esters ermöglicht oder daß sich durch Umesterung ein Ameisensäureester bildet. Einesteils wird der zweiten Möglichkeit in der Literatur widersprochen (Pruett, Kacmarcik, Organometallics 1 (1983) 1693). Insbesondere aber ist es aufgrund des bekannten Standes der Technik überraschend, daß eine Umsetzung nach Gleichung 1 ohne nennenswerte Nebenreaktionen abläuft. So beschreibt die DE-OS 16 18 547 (= US-PS 3 488 383) die edelmetallkatalysierte, schnelle Zersetzung von Ameisensäure im Gemisch mit Estern zumindest zum Teil auch zu $CO_2$ und $H_2$ schon bei vergleichsweise tiefer Temperatur von etwa 120 °C. Die EP-Appl. 60 695 bringt zum Ausdruck, daß bei höherem Umsatz gemäß Gleichung 5 Ameisensäure zersetzt wird. Im Vergleichsbeispiel c wird weiter unten deutlich, daß die Kinetik der Ameisensäurezersetzung unter den Bedingungen des erfindungsgemäßen Verfahrens von einer Art ist, die einen Ablauf der Reaktion nach Gleichung 1 über ein Freisetzen von Wasser aus der Ameisensäure und Verseifung des Esters ausschließt.

Das erfindungsgemäße Verfahren bietet nicht nur eine Möglichkeit zur Herstellung von Carbonsäuren, sondern kann auch benutzt werden zur Entfernung von beliebigen Mengen eines Esters oder alternativ von Ameisensäure aus einem Gemisch oder als eine Variante zur Esterverseifung, bei der weder Abfallsalze anfallen noch Wasser oder Alkohol abgetrennt werden müssen, wenn für die gleichzeitig entstehende zweite Carbonsäure ebenfalls Verwendung besteht. Die besonderen Vorteile des erfindungsgemäßen Verfahrens bestehen darin, daß die Ameisensäure in stöchiometrischer Menge eingesetzt und dadurch ein Verlust vermieden werden kann, während der Umsetzung kein nennenswerter Wassergehalt auftritt und das Produkt in praktisch wasserfreier Form anfällt. Das Verfahren wird im folgenden näher beschrieben.

Man bringt Ameisensäure und einen Ester $R^1COOR^2$ hinreichend lange, im allgemeinen 0,1 bis 10 Stunden, bei einer erhöhten Temperatur von 100 bis 300 °C mit einem Katalysatorsystem in Kontakt, welches Rhodium, ein Rhodiumsalz oder einen Rhodiumkomplex, ein Halogen als Promotor und eine zweite Metallkomponente in Form einer Verbindung eines Elementes der VI. Nebengruppe oder stattdessen bzw. zusätzlich einen Liganden aus der Gruppe der organischen Stickstoff- und Phosphorverbindungen enthält. Die Reaktion erfolgt in weitgehend wasserfreiem Medium.

Rhodium kann elementar in fein verteilter Form, als anorganisches oder organisches Salz oder als Komplexverbindung eingesetzt werden. Dabei ist es vorteilhaft, daß sich aus so einfachen Verbindungen wie $RhCl_3 \cdot xH_2O$, die eine ausgezeichnete Wirksamkeit für das Verfahren zeigen, unter Reaktionsbedingungen die aktive Katalysatorform ohne spezielle Präformierung schnell bildet. Weitere geeignete Einbringungsformen sind $Rh_2O_3$, $RhBr_3$, $RhJ_3$, Rhodium-(III)-acetylacetonat, Rhodium-(II)-acetat-Dimer, Chloro-dicarbonylrhodium-I-Dimer, Tetrarhodiumdodecacarbonyl und andere mehr. Geeignet sind auch Rhodiumkomplexe, die schon einen Stickstoff- oder Phosphorliganden enthalten, wie beispielsweise $Rh(Pyridin)_3Cl_3$, $RhCl(PPh_3)_3$, $RhCl(CO)(PPh_3)_2$ und andere mehr.

Als Halogenpromotoren eignen sich Jod und, mit schwächerer Promotorwirkung, Brom als Element oder in Form einer Verbindung. Geeignete Einsatzformen umfassen Verbindungen der Form $X_3^-$ und HX mit X = J oder Br, anorganische und organische Jodide und Bromide. Als anorganische Verbindungen können Salze derjenigen Metalle dienen, die die Reaktion ihrerseits nicht ungünstig beeinflussen, wie zum Beispiel Jodide und Bromide der Alkali- und Erdalkalimetalle oder von den Übergangsmetallen die des

Nickels oder auch der Katalysatormetalle selbst, als organische Verbindungen Alkyl-, Acyl- und Aryljodide und -bromide. Diese Verbindungen können einzeln oder im Gemisch miteinander eingesetzt werden. Geeignet sind auch Addukte einer organischen Jod- oder Bromverbindung mit dem Stickstoff- oder Phosphorliganden, wie beispielsweise ein Tetraalkylammonium- oder -phosphoniumjodid oder ein N-Alkylpyridiniumjodid. Um die erwünschten hohen Carbonsäureausbeuten zu erhalten, sollte jedoch nicht die gesamte Menge an Promotor in dieser gebundenen, sondern ein Teil noch in freier Form vorliegen.

Entsprechend sollten Promotor und Stickstoff- oder Phosphorverbindung in solchen Mengen eingesetzt werden, daß das Verhältnis von gesamter Menge an Jod- und Bromatomen zur gesamten Menge an Stickstoff- oder Phosphoratomen größer als 1 ist. Bevorzugt sind Jod und seine Verbindungen, insbesondere Methyljodid oder entsprechend dem Ausgangsester ein Jodid $R^1J$ oder $R^2J$.

Geeignete organische Stickstoffverbindungen gehören zu den Gruppen

(a) der heterocyclischen aromatischen Amine, wie beispielsweise Chinolin, Isochinolin und deren Derivate, Pyridin und dessen substituierte Derivate wie Picoline und Lutidine;
(b) der aromatischen Amine, wie beispielsweise die N.N-Dialkylaniline, N-Alkyldiphenylamine oder am aromatischen Ring substituierte Derivate derselben;
(c) der aliphatischen, cycloaliphatischen und araliphatischen Amine, wie beispielsweise Tri-n-alkylamine wie Triethyl- bis Tri-n-dodecylamin oder entsprechende Verbindungen mit ungleichen Alkylgruppen, verzweigte

Alkylamine wie das Tris-(2-ethylhexyl)amin oder das Tricyclohexylamin, N-Alkylpyrrolidine und -piperidine oder N-Alkyl-morpholine, weiterhin die entsprechenden primären und sekundären Amine;

(d) Carbonsäureamide wie Form- und Acetamid und ihre N-Alkyl- und N.N-Dialkylderivate sowie entsprechende Verbindungen höherer Carbonsäuren, zu denen beispielsweise auch aromatische Amide wie das Benzamid, N-Alkyl- und N.N-Dialkylbenzamide sowie Lactame wie Pyrrolidon, N-Alkylpyrrolidone, Piperidon und N-Alkylpiperidone gehören.

Bevorzugte organische Stickstoffverbindungen sind die heterocyclischen aromatischen Amine der Gruppe (a), die N.N-disubstituierten Aniline der Gruppe (b), die mit kurzen ($C_1$- bis $C_4$-)Alkylresten N-substituierten cycloaliphatischen Monoamine der Gruppe (c) und die N.N.-disubstituierten Carbonsäureamide der Gruppe (d). Bei den Verbindungen der Gruppe (d) sind die Form- und Acetamide sowie die den Produkten entsprechenden Amide $R^1CONH_2$ und $R^2CONH_2$ bzw. deren N-substituierten Derivate besonders bevorzugt, da mit ihnen dieselben Carbonsäurereste eingeführt werden, die schon zum Reaktionsgemisch gehören.

Geeignete organische Phosphorverbindungen sind tertiäre Phosphane der allgemeinen Form $R_3P$ und $R_2P(CH_2)_n\ PR_2$, wobei die Reste R gleiche oder verschiedene Alkylgruppen mit 1 bis 10 C-Atomen oder Phenyl-, substituierte Phenyl-, Phenylalkyl- oder Naphthylgruppen bedeuten und n = 1 bis 6 beträgt. Bei aliphatischen Resten R kann es sich um unverzweigte, verzweigte oder cyclische Substituenten handeln; beide Reste R an einem Phosphoratom können mit diesem auch einen Ring bilden. Bevorzugte Phosphorverbindung ist das Triphenylphosphan.

Bevorzugter Zusatz zum Rhodium-Halogen-Katalysator ist eine Verbindung eines Metalls der VI. Nebengruppe. Von diesen sind Chrom- und Molybdänverbindungen bevorzugt, insbesondere Chromverbindungen. Geeignete Einsatzformen umfassen die Carbonyle, Halogenide, Acetate, Acetylacetonate sowie andere organische oder anorganische Salze und Komplexe der Metalle, wobei sich die Bedingung von selbst versteht, daß derartige Verbindungen keine den Rhodiumkatalysator vergiftenden Bestandteile enthalten dürfen.

Zum Einsatz in das erfindungsgemäße Verfahren eignen sich Ester der allgemeinen Form $R^1COOR^2$. Den Carbonsäurerest $R^1$ und den Alkoholrest $R^2$ wählt man zweckmäßigerweise gleich, da dadurch die Aufarbeitung des Produktes einfacher ist; beide Reste können jedoch auch voneinander verschieden sein. Beispielsweise kann man vorteilhaft einen Alkoholrest $R^2$ höherer mit einem Carbonsäurerest niederer C-Zahl kombinieren und umgekehrt. Zusätzlich zu den Resten, die sich zugleich als Alkoholrest $R^2$ eignen, können als Carbonsäurereste $R^1$ auch Phenyl- und substituierte Phenylgruppen, beispielsweise mit einem oder mehreren Halogen-, Alkoxi-, Aryloxi-, Hydroxi-, Alkyl- oder Arylsubstituenten, Verwendung finden. Als Reste $R^2$ eignen sich (a) geradkettige oder verzweigte Alkylgruppen mit 1 bis 30 Kohlenstoffatomen, besonders Methyl, (b) Cycloalkylgruppen mit 3 bis 12 Kohlenstoffatomen und (c) Aralkylgruppen der Form $-C_nH_{2n}-Ar$, besonders $-CH_2-Ar$. Dabei bedeutet $C_nH_{2n}$ geradkettige oder verzweigte Alkylreste mit n gleich 1 bis 20 und Ar eine Phenyl- oder substituierte Phenylgruppe 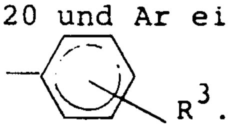. Der Rest $R^3$ steht für H oder (ca) einen beliebigen Alkylrest mit 1 bis 10 Kohlenstoffatomen, (cb) einen Alkoxirest $R^4O-$, wobei $R^4$ einen beliebigen Alkylrest mit 1 bis 10 Kohlenstoffatomen oder

oder eine Gruppe $R^5-(OCH_2CH_2)_n-$ mit $R^5 = C_1-$ bis $C_4-$Alkyl und n = 1 bis 3 bedeutet, (cc) eine Hydroxylgruppe oder (cd) ein Halogenatom. Der Einsatz von Verbindungen mit den Substituenten $R^1$ = Hydroxiphenyl oder $R^3$ = OH gibt durch Umesterung teilweise Oligomere. Ebenfalls einsetzbar sind bifunktionelle Verbindungen aus den Gruppen (a) und (b) der allgemeinen Form $(R^1COO)_2R^2$ oder $R^1(COOR^2)_2$, wobei die Summe der Kohlenstoffatome im ersten Fall 6 bis 30 und im zweiten 5 bis 30 beträgt und sich im ersten Fall die beiden Estergruppen nicht am gleichen Kohlenstoffatom befinden. Bei den Verbindungen der Gruppe (c) kann die Alkylkette $-C_nH_{2n-1}-$ ebenfalls zwei nicht geminale Esterfunktionen tragen; außerdem kann $R^2$ für Aralkylgruppen der Form $-C_mH_{2m}-Ar-C_pH_{2p}-$ stehen, wobei m + p = n gilt.

Das Verfahren eignet sich besonders für die Umsetzung von Methyl- und Benzylestern zu Essigsäure bzw. Phenylessigsäure und der der Ausgangsverbindung entsprechenden Carbonsäure $R^1COOH$. Zweckmäßigerweise setzt man Ester und Ameisensäure im äquivalenten Verhältnis ein. Eine geringere Menge an Ameisensäure ist unproblematisch, läßt allerdings eine entsprechende Menge des Esters unumgesetzt. Ein Ameisensäureüberschuß kann ebenfalls ohne weiteres angewendet werden, doch bewirkt dieser einen zusätzlichen Trennaufwand mit der Möglichkeit einer teilweisen Zersetzung der überschüssigen Ameisensäure. Insbesondere im Fall der Essigsäureherstellung, bei der überschüssige Ameisensäure besonders störend wäre, besteht der Vorteil des vorliegenden Verfahrens gerade darin, daß es praktisch wasser- und ameisensäurefreies Produkt liefert. Außer zur Produktion einer Carbonsäure läßt sich die Reaktion auch anwenden zur Entfernung von Ester- bzw. Ameisensäuregehalten aus einem Produktgemisch, wobei durch geeignete Wahl der Reaktionsbedingungen eine selektive Entfernung von Methylacetat aus einem Gemisch

von Estern möglich ist. Ist eine Carbonsäure $R^2COOH$ gleichzeitig ein gewünschtes Produkt, stellt das Verfahren auch eine Alternative zur konventionellen Esterverseifung dar, bei der Salzanfall bzw. ein Wassereinsatz in eventuell überschüssiger, anschließend wieder abzutrennender Menge bzw. Probleme mit der während der Umsetzung erforderlichen Abtrennung des entstehenden Alkohols vom Ester vermieden werden.

Die Umsetzung erfolgt bevorzugt in der Flüssigphase. Die Verwendung eines Lösemittels ist nicht erforderlich, da die Reaktion im flüssigen oder geschmolzenen Ausgangsmaterial bzw. Produkt glatt abläuft. Falls aus technischen Gründen die Verwendung eines Lösemittels erwünscht sein sollte, können solche ohne weiteres angewendet werden. Beispiele dafür sind andere Carbonsäuren, Ketone und Ether, wobei die Auswahl einer speziellen Verbindung selbstverständlich abhängt von der Möglichkeit zur späteren Abtrennung vom Produkt. Alkohole oder andere Ester sind als Lösemittel deshalb unzweckmäßig, weil sie gleichfalls durch katalytische Umsetzung Carbonsäuren bilden.

Es ist vorteilhaft, die Umsetzung in praktisch wasserfreiem Medium durchzuführen, um auf einfache Weise absolutes Produkt zu erhalten, die Korrosivität des Mediums zu vermindern und unerwünschte Nebenreaktionen, die in Gegenwart größerer Wassermengen deutlich zunehmen, zu vermeiden. Geringe Wassergehalte von nicht mehr als 5 Gewichtsprozent, günstiger von micht mehr als 2 Gewichtsprozent, können jedoch geduldet werden. Auch stören Verunreinigungen, wie beispielsweise die den eingesetzten Estern entsprechenden Alkohole $R^2OH$ nicht. Sie werden ebenfalls zur Carbonsäure $R^2COOH$ umgesetzt.

Die Katalysatorkomponenten werden vorzugsweise in der Flüssigphase suspendiert oder homogen gelöst, wobei die Metallkomponenten auch auf Trägern wie Aktivkohle, Aluminiumoxid oder Kieselgel aufgebracht sein können. Das Verfahren läßt sich kontinuierlich oder diskontinuierlich durchführen.

Nach beendeter Umsetzung kann das Produkt destillativ abgetrennt werden; flüchtige Jod- bzw. Bromverbindungen werden dabei als Vorlauf abgenommen und gemeinsam mit dem bei Durchführung in homogener Phase den Katalysator enthaltenden Destillationssumpf in den Reaktor zurückgeführt. Je nach Art der verwendeten Liganden werden diese mit dem Katalysator oder als eine Destillationsfraktion ebenfalls in den Prozeß zurückgeführt.

Das erfindungsgemäße Verfahren ist jedoch auf eine bestimmte Ausprägung der technischen Ausführung nicht beschränkt.

Die Reaktion erfolgt beim Dampfdruck der beteiligten Verbindungen oder in Gegenwart eines Inertgases. Bevorzugt ist die Durchführung der Reaktion in einer Kohlenmonoxidatmosphäre. Diese wird zwar gemäß der Stöchiometrie für die Umsetzung nicht benötigt und ist insofern als Inertgas anzusehen, dient aber zur Stabilisierung des Ameisensäureesters und der aktiven Katalysatorform gegen Zersetzung und fördert nötigenfalls die Bildung dieser aktiven Katalysatorform aus den eingesetzten Verbindungen. Auf diese Weise läßt sich die Aktivität, Selektivität und Lebensdauer des Katalysators verbessern. Die Gasphase kann, gegebenenfalls unter Abzweigen eines kleinen Abfallstroms zum Ausschleusen gasförmiger Verunreinigungen, im Kreis geführt werden. Kohlenmonoxid kann Wasserstoff, Stickstoff, Methan, Kohlendioxid oder andere inerte Gase auch in

größerer Menge enthalten; beispielsweise kann das Verfahren unter Synthesegasdruck oder in einer Stickstoffatmosphäre durchgeführt werden. Bei der bevorzugten Arbeitsweise in Gegenwart von Kohlenmonoxid sollten Fremdgase aber gering gehalten werden, um den Reaktionsdruck möglichst niedrig zu halten. Bei Verwendung von reinem Kohlenmonoxid führt man die Reaktion bei CO-Kaltdrücken von 1 bis 250 bar, bevorzugt bei 3 bis 100 bar, insbesondere nicht mehr als 50 bar, durch. Die Reaktionsdrücke stellen sich zwangsläufig in Abhängigkeit von der Zusammensetzung der flüssigen Reaktionsmischung und der Reaktionstemperatur ein; im bevorzugten Bereich der Temperatur und dem besonders bevorzugten des Kaltdrucks betragen sie zwischen rund 10 und 80 bar und können ohne weiteres im Bereich von 15 bis 50 bar gehalten werden. Verwendet man ein anderes, inertes Gas, vor allem Stickstoff anstelle des Kohlenmonoxids, ist der Bereich der Kalt- und Reaktionsdrücke der gleiche; zweckmäßig ist es in diesem Fall, im untersten Druckbereich zu arbeiten.

Die Umsetzungstemperatur des Verfahrens beträgt 100 bis 300 °C, bevorzugt 160 bis 220 °C. Die Reaktionsdauer kann in Abhängigkeit von den übrigen Verfahrensparametern in weiten Grenzen variieren und wird zweckmäßigerweise so eingerichtet, daß ein praktisch vollständiger Umsatz erreicht wird, im allgemeinen in 0,1 bis 10 Stunden. Eine Reaktionsdauer von zwei Stunden und weniger läßt sich dabei ohne weiteres erreichen.

Es ist leicht einzusehen, daß der Einsatz einer möglichst geringen Menge an Katalysator, vor allem des Rhodiums als dem kostspieligsten Bestandteil, wirtschaftlich wünschenswert ist. Daher ist es von Vorteil, daß das Verfahren mit sehr geringem Katalysatoreinsatz auskommt.

Geeignet sind Mengen an Rhodium(verbindung), die 0,05 bis 5 mg-atom Metall pro mol Carbonsäureester, bevorzugt 0,1 bis 2 mg-atom/mol, entsprechen. Dabei beträgt das Atomverhältnis Rhodium zu Halogen, bevorzugt Rh/J, 1 : 1 000 bis 1 : 1, bevorzugt 1 : 100 bis 1 : 5. Das Atomverhältnis Rhodium zu Verbindung der VI. Nebengruppe , bevorzugt Rh/Cr, beträgt 1 : 100 bis 10 : 1, vorzugsweise 1 : 20 bis 2 : 1. Setzt man einen der als zusätzliche Komponente weniger bevorzugten Liganden aus der Gruppe der organischen Stickstoff- oder Phosphorverbindungen ein, so beträgt das Verhältnis zum Rhodium, ausgedrückt als Atomverhältnis Rh/N bzw. Rh/P, 1 : 100 bis 1 : 1, bevorzugt 1 : 40 bis 1 : 2, wobei es nicht erforderlich ist, die organische Stickstoffverbindung als Lösemittel in Mengen von mindestens 0,2 mol/mol Ester einzusetzen. Es wird ein Verhältnis des J oder Br zu N bzw. P von größer als 1 eingehalten, um besonders gute Ergebnisse zu erzielen. Die drei Typen zusätzlicher Komponenten können sowohl einzeln als auch im Gemisch miteinander eingesetzt werden. Man kann zwar grundsätzlich auch größere Mengen jeder Komponente anwenden, jedoch bewirkt das keine Verbesserung und ist wirtschaftlich ungünstig. Bei kleineren Mengen dagegen ist es notwendig, in unerwünschter Weise die Umsetzungsdauer zu verlängern oder die Reaktionsbedingungen zu verschärfen. Es ist vorteilhaft, die Ameisensäure in äquivolarer Menge zum Ester $R^1COOR^2$ einzusetzen. Kleinere Einsatzmengen sind anwendbar, führen aber naturgemäß zu unvollständigem Esterumsatz. Größere Ameisensäuremengen können gleichfalls eingesetzt werden, wenngleich dabei Ameisensäureverluste eintreten können.

Durch die folgenden Beispiele wird das Verfahren weiter erläutert.

Beispiel 1

In einem 100 ml-Hubrührautoklaven aus Hastelloy C werden 15,7 g (341 mmol) Ameisensäure, 25,3 g (341 mmol) Methylacetat, 0,2 g (0,76 mmol) $RhCl_3 \cdot 3\ H_2O$, 0,8 g (3,6 mmol) $Cr(CO)_6$ und 5 g (35,2 mmol) Methyljodid vorgelegt. Der Autoklav wird druckfest verschlossen, zweimal mit 10 bar Kohlenmonoxid gespült, dann mit Kohlenmonoxid auf 30 bar gebracht und auf 180 °C aufgeheizt. Bei dieser Temperatur wird der Reaktionsdruck auf 50 bar eingestellt und die Umsetzung 1,5 Stunden lang durchgeführt. Anschließend entspannt man über einen Abgaswäscher und entnimmt den Autoklaveninhalt nach Zusatz einer definierten Menge 1,4-Dioxan, das als innerer Standard dient. Austrag, Wäscherflüssigkeit und das aufgefangene Abgas werden gaschromatographisch analysiert. Das Abgas enthält je 2 mmol Wasserstoff und $CH_4$. Beide Ausgangsverbindungen sind vollständig umgesetzt; das Flüssigprodukt (Austrag und Wäscherinhalt) besteht ausschließlich aus Essigsäure, deren Selektivität bezüglich beider Edukte über 99 % beträgt. Die Essigsäure enthält eine Spur Wasser (0,6 Gew.-%), die fast völlig aus den Einsatzmaterialien stammt.

Beispiele 2 und 3

Beispiel 1 wird wiederholt, indem man als Rhodiumverbindung eine äquivalente Menge $[Rh(CO)_2Cl]_2$ bzw. $Rh_4(CO)_{12}$ einsetzt. Die Versuchsergebnisse sind praktisch identisch.

Beispiel 4

Beispiel 1 wird wiederholt, wobei man statt des $Cr(CO)_6$ 1,6 g (20 mmol) Pyridin einsetzt. Der Umsatz ist $>$ 99,7 %. Neben einer Spur unumgesetztem Methylacetat ($<$ 1 mmol) zeigt die Analyse nur Essigsäure.

Beispiel 5

Beispiel 1 wird wiederholt, wobei man statt des $Cr(CO)_6$ 0,4 g (1,5 mmol) Triphenylphosphan einsetzt. Man erhält eine quantitative Essigsäureausbeute.

Beispiel 6

Beispiel 1 wird wiederholt, wobei man als Jodpromotor 5,98 g (35,2 mmol) $LiJ \cdot 2\ H_2O$ verwendet. Das Produktgemisch enthält jeweils maximal 1 mmol Methylformiat, Methylacetat und Methanol; im Abgas finden sich 5 mmol $H_2$ und 1 mmol Methan. Der Umsatz beträgt demnach $>$ 99,7 % und die Selektivität für Essigsäure $>$ 99 %.

Beispiel 7

Einem Ansatz gemäß Beispiel 1 wird 1 g (55,5 mmol) Wasser zugesetzt und die Reaktion wie zuvor durchgeführt. Man ermittelt anschließend einen vollständigen Umsatz und eine Essigsäureselektivität von mindestens 97 %. Das Beispiel zeigt, daß ein kleiner Wassergehalt, der hier knapp 2,5 Gew.-% des organischen Einsatzes beträgt, nicht stört.

Vergleichsbeispiel a

Beispiel 7 wird mit einem Wasserzusatz von 5 g, entsprechend 12,2 Gew.-% des organischen Einsatzes, wiederholt. Während der Reaktion steigt der Druck von 50 auf 80 bar. Das Abgas enthält 19 mmol $H_2$, 4 mmol $CH_4$ und eine damit korrespondierende Menge von 23 mmol $CO_2$. Im flüssigen Austrag werden nachgewiesen: $<$ 1 mmol Methylformiat, 2 mmol Methylacetat, 16 mmol Ameisensäure, 656 mmol Essigsäure und 251 mmol Wasser. Der Versuch zeigt, daß ein größerer Wassergehalt eine Nebenreaktion gemäß

$CH_3COOCH_3 + H_2O + CO \rightarrow 2\ CH_3COOH$ bewirkt. Dadurch wird die Ameisensäure unvollständig verbraucht, so daß man ein schwierig zu trennendes Gemisch von Wasser, Essig- und Ameisensäure als Produkt erhält. Außerdem erfolgt vor allem bei der Ameisensäure eine merkliche Zersetzung zu $H_2 + CO_2$.

Beispiel 8

Beispiel 1 wird wiederholt, wobei man als Jodpromotor 6,72 g einer 67 gew.-%igen wäßrigen HJ-Lösung verwendet. Der Umsatz beträgt 99,9 %. Neben Essigsäure werden jeweils wenige Zehntel mmol Methylformiat und Methanol nachgewiesen; die Essigsäure-Selektivität beträgt mindestens 99,5 %. In diesem Fall wirkt sich der Wassergehalt im Einsatz noch nicht nachteilig aus.

Beispiel 9

Beispiel 1 wird wiederholt, wobei man jedoch nur einen CO-Kaltdruck von 5 bar aufpreßt. Bei der Reaktionstemperatur stellt sich ein Heißdruck von 19 bar ein. Das Produktgemisch enthält: 0,1 mmol Methylformiat, 1,5 mmol Methylacetat, 1 mmol Methanol und 678 mmol Essigsäure; Ameisensäure ist nicht mehr nachzuweisen. Das bedeutet einen Umsatz von 99,5 bzw. 100 % und eine Ausbeute für Essigsäure von 99,4 %. Demnach ist es möglich, schon durch einen geringen CO-Druck die Umsetzung praktisch ohne Zersetzung der Edukte zu führen.

Beispiel 10

Beispiel 9 wird wiederholt, wobei die Gasphase im kalten Autoklaven aus Stickstoff unter Normaldruck besteht. Der Heißdruck stellt sich auf 15 bar ein.

Nach 90minütiger Reaktion enthält das Abgas 1 bis 2 mmol $H_2$, 5 bis 6 mmol $CH_4$, 4 bis 5 mmol CO und 6 bis 7 mmol $CO_2$. Das flüssige Reaktionsgemisch setzt sich zusammen aus 457 mmol Essigsäure, 102 mmol Methylacetat, 103 mmol Ameisensäure, 10 mmol Methylformiat, $<$ 1 mmol Methanol und 6 mmol Wasser; die geringfügigen Abweichungen von der C-Bilanz gemäß Reaktionstöchiometrie beruhen auf analytischen Ungenauigkeiten. Man berechnet daraus den Umsatz zu 70 % und die Essigsäureselektivität zu 96 %; berücksichtigt man, daß im weiteren Reaktionsverlauf auch aus Methylformiat und Methanol Essigsäure entsteht, beträgt die Selektivität sogar 98 %.

Das Beispiel zeigt, daß ein CO-Kaltdruck für die erfindungsgemäße Umsetzung nicht erforderlich ist. Im direkten Vergleich mit Beispiel 1 oder 9 erkennt man eine mäßig langsamere Reaktionsgeschwindigkeit und eine leicht verminderte Selektivität, wobei jedoch zu Vergleichszwecken hier weder das Katalysatorsystem noch die physikalischen Bedingungen auf maximale Selektivität optimiert wurden. Das Beispiel widerlegt das in der EP-PS 45 637 geäußerte Vorurteil, daß ein Rhodiumkatalysator die Umsetzung überhaupt nicht bewirkt, wenn kein CO-Druck angewendet wird. Der Vergleich mit den experimentellen Beispielen der EP-PS 45 637 zeigt im Gegenteil, daß das erfindungsgemäße Rhodium-Katalysatorsystem für die Umsetzung auch dann mindestens so aktiv ist wie der dort angewendete Iridiumkatalysator.

<u>Beispiele 11 und 12</u>

Beispiel 1 wird bei 160 und 170 °C wiederholt. Während nach 1,5 Stunden bei 170 °C die Zusammensetzung von flüssigem Austrag und Abgas praktisch identisch mit der im Beispiel 1 ist, beträgt bei 160 °C zu diesem Zeitpunkt der Umsatz erst 63 % und die Essigsäureselektivität 87 % neben 13 % Methylformiat (berechnet als C-Atom-%, bezogen auf umgesetztes Methylacetat und Ameisensäure). Nach einer weiteren Stunde ist der Umsatz des Methylacetats und der Ameisensäure vollständig. Um das dann noch vorhandene Umesterungsprodukt Methylformiat, das bei dieser Temperatur vergleichsweise langsam abreagiert, ebenfalls in Essigsäure zu überführen, wird die Temperatur für 30 Minuten auf 170 °C angehoben.
Danach ist die Essigsäureausbeute praktisch quantitativ.

Beispiel 13

Der Autoklav aus Beispiel 1 wird mit 30 g (500 mmol) Essigsäure, 7,4 g (100 mmol) Methylacetat, 1,4 g (30 mmol) Ameisensäure, 0,2 g $RhCl_3 \cdot 3\ H_2O$, 0,8 g $Cr(CO)_6$ und 5 g Methyljodid beschickt und mit $N_2$ gespült. Beim Aufheizen auf 173 °C steigt der Druck von 1 auf 16,5 bar; 30 Minuten später beträgt er bei 180 °C 18,5 bar und weitere 15 Minuten später wird die Umsetzung beendet. Die Analyse des Austrags ergibt: 570 mmol Essigsäure, 65 mmol Methylacetat, keine Ameisensäure, 14 mmol Wasser, davon ein Teil aus der Zersetzung von Ameisensäure, sowie im Abgas 7 mmol CO.
Der Versuch beschreibt die Möglichkeit, Ameisensäure in Gegenwart eines Esters aus einem Gemisch zu entfernen und dabei gleichzeitig den Ester zur Carbonsäure umzusetzen. Im vorliegenden Beispiel erfolgt ein Teil des Esterumsatzes mit dem im nicht absolutierten Einsatz vorhandenen Wasser.

Beispiel 14

Die Umsetzung erfolgt wie in Beispiel 1, jedoch mit der halben Menge aller drei Katalysatorkomponenten und bei 200 °C. Der Umsatz beträgt $>$ 99,5 %; neben

Essigsäure sind 0,5 mmol Methanol und <0,5 mmol Methylformiat nachweisbar.

Beispiel 15

Der Autoklav wird mit 15,7 g Ameisensäure, 25,3 g Methylacetat, 30 mg $RhCl_3 \cdot 3\ H_2O$, 0,2 g $Cr(CO)_6$ und 5 g Methyljodid beschickt und die Umsetzung unter CO bei 200 °C und 50 bar durchgeführt. Nach 1,5 Stunden sind 93 % Umsatz erreicht; die Essigsäureselektivität beträgt 98,9 %.
Als Nebenprodukte werden 7 mmol Methylformiat und 0,5 mmol Methanol nachgewiesen, die ebenfalls in Essigsäure überführbar sind.

Beispiel 16

Beispiel 14 wird wiederholt. Nach nur 60minütiger Reaktion statt zuvor 1,5 Stunden beträgt der Umsatz 88 % und die Selektivität für Essigsäure 97 %; die restlichen 3 C-Atom-% bestehen aus Methylformiat.

Beispiele 17 bis 19

Beispiel 14 wird wiederholt, wobei als Chromkomponente jeweils 1,8 mmol Chrom-(III)-chlorid-hexahydrat, Chrom-(III)-acetylacetonat oder Chrom-(III)-acetat eingesetzt werden. In allen Fällen liegt der Umsatz bei > 95 bis fast 100 % und es werden höchstens Spuren an Methylformiat, Methanol, Methan oder $H_2$ neben der Essigsäure gefunden.

Beispiel 20

Beispiel 14 wird wiederholt, wobei zusätzlich 6 g Essigsäure im Einsatz vorhanden sind. Nach nur 30 Minuten bei 200 °C und 50 bar erreicht der Umsatz

70 % und die Selektivität zu neugebildeter Essigsäure 96,4 %; daneben sind 17 mmol Methylformiat, entsprechend 3,5 % Selektivität, vorhanden. Das Beispiel zeigt, daß die Gegenwart einer zweiten Carbonsäure im Einsatz sich nicht nachteilig auswirkt.

Vergleichsbeispiel b

Beispiel 14 wird wiederholt, indem man den Rhodiumkatalysator durch 0,38 mmol $IrCl_3 \cdot 3\ H_2O$ ersetzt. Unter sonst gleichen Bedingungen beträgt der Umsatz des Methylacetats nur 66 % und der der Ameisensäure 69 %. Die Selektivität für Essigsäure beträgt 90,9 % und für Methylformiat 3,5 % (C-Atom-%, bezogen auf Methylacetat und Ameisensäure).
Der Rest geht durch Zersetzung zu $H_2$, $CH_4$ und $CO_2$ verloren.

Vergleichsbeispiele c bis e

15,3 g (332 mmol) Ameisensäure, 20,5 g (341 mmol) Essigsäure und die gleichen Mengen an Katalysatorkomponenten wie in Beispiel 15 werden unter 1 bar $N_2$ zusammengegeben und auf 200 °C aufgeheizt. Bei Erreichen der Temperatur beträgt der Druck 96 bar, nach 30 Minuten bei dieser Temperatur 101 bar und nach 90 Minuten 105,5 bar. Anschließend wird abgekühlt. Der Kaltdruck beträgt dann noch 69 bar; das Abgas besteht neben dem ursprünglich vorhandenen $N_2$ praktisch nur aus CO. Titration des flüssigen Inhalts ergibt 14 mmol restliche Ameisensäure. Führt man den Versuch ausgehend von 50 bar CO-Kaltdruck durch, ist keine Verlangsamung der Gasentwicklung zu erkennen.

Heizt man nur bis auf 180 °C auf, so beobachtet man folgende Drücke: 46 bar bei Erreichen der Temperatur, 90,5 bar 45 Minuten später, 95,5 bar nach weiteren 45 Minuten; Rest Ameisensäure 36 mmol. Der beobachtete Druckverlauf zeigt an, daß in Gegenwart des erfindungsgemäßen Katalysators ein großer bis überwiegender Teil der Ameisensäure schon in der Aufheizphase zersetzt wird, wogegen die Zersetzungsgeschwindigkeit des Restes weitaus geringer ist. Diese Beobachtung steht im Gegensatz zum Reaktionsablauf der entsprechenden Umsetzung von Ameisensäure mit Methylacetat.

($\alpha$) Sie ist unterhalb 200 °C beim obigen Katalysatoreinsatz nur langsam; bei nur 180 °C bildet sich bei Durchführung gemäß Beispiel 15 Essigsäure nur in 15 %iger Ausbeute, zudem zu über zwei Dritteln durch bloße Umesterung.

($\beta$) Dabei liegt die noch nicht verbrauchte Ameisensäure ebenso in unzersetzter Form vor wie bei unvollständigen Reaktionen bei 200 °C.

($\gamma$) Sie ist im Anfangsstadium zumindest nicht schneller, wie die Beendigung einer Umsetzung gemäß Beispiel 16 nach 30 Minuten zeigt, und gegen Ende nicht wesentlich langsamer als im Durchschnitt über die gesamte Reaktionsdauer.

Da bei der erfindungsgemäßen Reaktion ein starker Druckanstieg zu Beginn der Umsetzung ausbleibt, kann ein Verlauf über eine Ameisensäurezersetzung zu CO und Wasser mit nachfolgender Esterverseifung und Alkoholcarbonylierung ausgeschlossen werden.

Vergleichsbeispiel f

Führt man die Umsetzung entsprechend Beispiel 1, jedoch ohne die Chromkomponente durch, ermittelt man einen Umsatz von nur 69 %, der teilweise auf bloße Umesterung zurückzuführen ist. Entsprechend beträgt die Selektivität für Essigsäure 89 % und für Methylformiat 11 %.
Man erkennt, daß ein nur aus einer Rhodium- und einer Jodkomponente bestehender Katalysator weniger wirksam ist als das erfindungsgemäße Katalysatorsystem.

Vergleichsbeispiele g bis i

Führt man die Umsetzung gemäß Beispiel 1 durch, jedoch unter Weglassen der Jod-, der Jod- und Chrom- bzw. aller drei Katalysatorkomponenten, ergibt die anschließende Analyse, daß etwas Essigsäure (65 bis 110 mol) nur durch Umesterung entstanden ist.

Vergleichsbeispiel j

Beispiel 10 wird wiederholt, jedoch mit einem Einsatz von 41 g (682 mmol) Methylformiat anstelle des äquimolaren Gemisches aus Methylacetat und Ameisensäure. Nach 1,5 Stunden beträgt der Umsatz nur um 10 %. Essigsäure ist nur in sehr geringer Menge vorhanden (ca. 2 mmol); daneben finden sich Methanol und Methylacetat sowie im Abgas $H_2$, $CH_4$, $CO_2$ und vor allem CO. Dieser Versuch zeigt, daß die Reaktion von Methylacetat mit Ameisensäure zu Essigsäure (gemäß Gl. 1) nicht über die Umesterung zu Methylformiat und dessen, wie auch immer geartete, Umlagerung zu Essigsäure verlaufen kann.

Beispiel 21

Beispiel 1 wird mit derselben Stoffmenge Methylpropionat anstelle des Methylacetats wiederholt. Der Umsatz ist vollständig; außer einer Spur Methylformiat ( < 1 mmol) findet man ausschließlich die erwarteten Mengen an Essig- und Propionsäure.

Beispiel 22

Beispiel 1 wird mit Methyl-n-butyrat durchgeführt. Der Umsatz beträgt 98,5 %. Man erhält die zu erwartende Menge an Essigsäure und Buttersäure.

Beispiel 23

Beispiel 1 wird mit Methyl-isobutyrat durchgeführt. Der Umsatz beträgt 74 %, die Essigsäureselektivität 90,5 %. Vorhanden sind außerdem Methanol, Methylformiat und Methylacetat, die ebenfalls zu Essigsäure weiterreagieren. Der Gehalt an Isobuttersäure entspricht der Reaktionsstöchiometrie.

Beispiel 24

Die Umsetzung wird in gleicher Weise mit Methylbenzoat durchgeführt. Der Umsatz beträgt 97 %, man stellt, abgesehen von einer geringen Menge Methanol, vollständige Selektivität der Umsetzung zu Essig- und Benzoesäure fest.

Beispiel 25

Die Umsetzung wird mit Phenylessigsäuremethylester wiederholt. Man erhält ganz entsprechend das Gemisch von Essig- und Phenylessigsäure.

Beispiel 26

Als Ester werden 51,2 g (341 mmol) Benzylacetat eingesetzt. Unter den obigen Standardbedingungen beträgt der Umsatz des Benzylacetats 75 % und der Ameisensäure 65 %. Bezogen auf umgesetzte Benzylgruppen werden die folgenden Selektivitäten ermittelt: 64,7 % Phenylessigsäure, 18,8 % Phenylessigsäurebenzylester, 8,2 % Benzylformiat, 2 % Benzaldehyd und 6,2 % Toluol. Die Selektivitätssumme für Phenylessigsäure und in diese überführbare Benzylverbindungen beträgt 91,7 %. Die Essigsäuremenge entspricht der Bilanz gemäß Reaktionsstöchiometrie.

Beispiel 27

Unter den Bedingungen des Beispiels 1 werden 48,8 g (205 mmol) Phenylessigsäurebenzylester (95 %ig) mit 9,45 g (205 mmol) Ameisensäure in Gegenwart von 0,12 g (0,46 mmol) $RhCl_3 \cdot 3\ H_2O$, 0,48 g (2,2 mmol) $Cr(CO)_6$ und 3 g (21,1 mmol) Methyljodid umgesetzt. Man erhält einen Umsatz von 67 %. Das Reaktionsgemisch enthält, in Selektivitäten bezogen auf umgesetzte Benzylgruppen: 81,5 % Phenylessigsäure (90,9 % einschl. der im Ausgangsester vorhandenen $PhCH_2COO$-Gruppen), 8,7 % Toluol, 2,9 % Benzaldehyd, 5,8 % Benzylformiat, 0,2 % Benzylalkohol und 0,7 % Benzylacetat (durch Reaktion mit Methyljodid).

Beispiel 28

Wie in Beispiel 27 werden 51 g (239 mmol) Benzoesäurebenzylester mit proportional ebenfalls vermehrten Mengen der übrigen Einsatzkomponenten umgesetzt. Der Umsatz beträgt 77 %. Die Produktzusammensetzung ist ganz ähnlich wie bei den vorherigen Benzylestern mit Benzoesäure als der zweiten entstandenen Säure.

Beispiel 29

Entsprechend Beispiel 1 werden 30 g (341 mmol) Ethylacetat umgesetzt. Bei einem Umsatz von 49 % wurden folgende Produktselektivitäten ermittelt: 41 % Propionsäure, 2,5 % Ethylpropionat, 56 % Ethylformiat. Die Essigsäuremenge entspricht der Reaktionsbilanz.

Vergleichsbeispiel k

Setzt man unter den Bedingungen des Beispiels 29 Ethylformiat als alleiniges Edukt ein, erfolgt praktisch keine Reaktion.

Beispiel 30

Entsprechend Beispiel 1 werden 34,8 g (341 mmol) Isopropylacetat umgesetzt. Der Umsatz beträgt rund 60 %. Das Produktgemisch enthält n- und iso-Buttersäure im Verhältnis 2,3 : 1 sowie n- und iso-Propylformiat, Propanol und das Koppelprodukt Essigsäure.

Beispiel 31

Entsprechend Beispiel 1 werden 39,6 g (341 mmol) t-Butylacetat umgesetzt. Der Umsatz ist vollständig. Das Produktgemisch enthält n-Valerian- sowie 2- und 3-Methylbuttersäure entsprechend einer Ausbeute an $C_5$-Säuren von 25 % neben der theoretischen Menge von 20,5 g Essigsäure, unumgesetzter Ameisensäure und weiteren Komponenten; Isobuten wurde ebenfalls nachgewiesen.

Beispiel 32

Entsprechend Beispiel 1 werden 34,8 g (341 mmol) Ethylpropionat umgesetzt. Der Umsatz beträgt 63 %. Die Produktselektivitäten sind wie folgt: 69 % Propionsäure, 21,6 % Ethylformiat, 1,2 % Ethylacetat.

Das Ethylacetat stammt wie die ebenfalls nachgewiesenen 30 mmol Essigsäure aus dem Methyljodid; als organische Jodverbindung liegt nach der Umsetzung nur Ethyljodid vor (8,1 %, bezogen auf umgesetztes $C_2H_5$-). Die unumgesetzte Ameisensäuremenge entspricht der Reaktionsbilanz.

Vergleichsbeispiel 1

Entsprechend Beispiel 1 werden 51,2 g (341 mmol) p-Kresylacetat umgesetzt. Der Umsatz beträgt 89 %, das Produkt besteht fast ausschließlich aus Essigsäure und p-Kresol. Während der Umsetzung tritt keine nennenswerte Druckerhöhung auf. Das Beispiel verdeutlicht zwar die Möglichkeit der Esterverseifung mit Hilfe von Ameisensäure, ist jedoch insofern kein Beispiel gemäß der vorliegenden Erfindung, als keine p-Tolylsäure entsteht. Es beweist andererseits, daß Ester mit freien phenolischen Hydroxylfunktionen mit Ameisensäure erfindungsgemäß umsetzbar sind, da sich unter diesen Bedingungen stabile Phenylformiate nicht bilden.

F

Patentansprüche:

1. Verfahren zur Herstellung von Carbonsäuren durch Umsetzung von Carbonsäureestern der allgemeinen Formel

$$R^1COOR^2,$$

in der $R^2$ = (a) eine geradkettige oder verzweigte Alkylgruppe mit 1 bis 30 Kohlenstoffatomen, (b) eine Cycloalkylgruppe mit 3 bis 12 Kohlenstoffatomen und (c) eine Aralkylgruppe der Form $-C_n H_{2n}-Ar$. ($C_nH_{2n}$ bedeutet geradkettige oder verzweigte Alkylreste mit n gleich 1 bis 20 und Ar eine Phenyl- oder substituierte Phenylgruppe 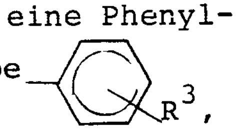,

der Rest $R^3$ steht für H oder (ca) einen beliebigen Alkylrest mit 1 bis 10 Kohlenstoffatomen, (cb) einen Alkoxirest $R^4O-$, wobei $R^4$ einen beliebigen Alkylrest mit 1 bis 10 Kohlenstoffatomen oder eine Gruppe $R^5-(OCH_2CH_2)_n-$ mit $R^5 = C_1$ bis $C_4$-Alkyl und n = 1 bis 3 bedeutet, (cc) eine Hydroxylgruppe oder (cd) ein Halogenatom) und $R^1$ = eine der gleichen Gruppen wie für $R^2$, zusätzlich auch eine Phenyl- und substituierte Phenylgruppe ist, mit Ameisensäure in Gegenwart eines Metallkatalysators und eines Halogenpromotors bei erhöhter Temperatur,
dadurch gekennzeichnet,
daß man die Umsetzung in der Flüssigphase an einem Metallkatalysatorsystem aus Rhodium bzw. Rhodiumsalzen oder Rhodiumkomplexen, einem Halogen oder einer Halogenverbindung als Promotor, einem Salz oder einem Komplex eines Elementes der VI. Nebengruppe und/oder Liganden aus der Gruppe der organischen Stickstoff- oder Phosphorverbindungen bei Temperaturen von 100 bis 300 °C und Kaltdrücken von 1 bis 250 bar durchführt, wobei man das Rhodium oder die Rhodiumverbindung in Mengen von 0,05 bis 5 mg-atom Rhodium

pro mol eingesetzten Carbonsäureester einsetzt und ein Atomverhältnis Rhodium : Halogen von 1 : 1 000 bis 1 : 1, ein Atomverhältnis Rhodium : Metall der VI. Nebengruppe von 1 : 100 bis 10 : 1 sowie gegebenenfalls ein Atomverhältnis Rh : N oder P von 1 : 100 bis 1 : 1 einhält.

2. Verfahren nach Anspruch 1, dadurch gekennzeichnet, daß man als Halogenpromotoren Jod, Brom oder Verbindungen von Jod und/oder Brom, bevorzugt Methyljodid, $R^1J$ oder $R^2J$ einsetzt.

3. Verfahren nach Anspruch 1 und 2, dadurch gekennzeichnet, daß man als Metallverbindung der VI. Nebengruppe Chrom- und/oder Molybdänverbindungen, vorzugsweise Chromverbindungen wie Chromcarbonyl, Chromacetat und/oder Chromhalogenid einsetzt.

4. Verfahren nach Anspruch 1 und 2, dadurch gekennzeichnet, daß man als organische Stickstoffverbindung heterocyclische aromatische, aromatische, aliphatische, cycloaliphatische und araliphatische Amine und/oder Carbonsäureamide einsetzt, bevorzugt heterocyclische aromatische Amine, N.N-disubstituierte Aniline und N-substituierte cycloaliphatische Monoamine, insbesondere mit $C_1$- bis $C_4$-Substituenten am N-Atom, sowie N.N-disubstituierte Carbonsäureamide, insbesondere Formamide und Acetamide oder die den Gruppen $R^1$ und $R^2$ entsprechenden Amide.

5. Verfahren nach Anspruch 1 und 2, dadurch gekennzeichnet, daß man als organische Phosphorverbindung Phosphane der allgemeinen Formel $R_3P$ und $R_2P(CH_2)_nPR_2$, in der R gleiche oder verschiedene Alkylgruppen mit 1 bis 10 C-Atomen oder Phenyl-, substituierte Phenyl-, Phenylalkyl- oder Naphthylgruppen bedeutet und n = 1 bis 6 ist, einsetzt, vorzugsweise Triphenylphosphan.

6. Verfahren nach Anspruch 1 bis 5, dadurch gekennzeichnet, daß man das Rhodium bzw. die Rhodiumverbindung in Mengen von 0,1 bis 2 mg-atom Rhodium pro mol eingesetzten Carbonsäureester einsetzt.

7. Verfahren nach Anspruch 1 bis 6, dadurch gekennzeichnet, daß man in dem Metallkatalysatorsystem ein Atomverhältnis Rhodium : Halogen von 1 : 100 bis 1 : 5, Rhodium : Metall der VI. Nebengruppe von 1 : 20 bis 2 : 1 und gegebenenfalls Rh : N oder P von 1 : 40 bis 1 : 2 einhält.

8. Verfahren nach Anspruch 1, 2, 4 und 5, dadurch gekennzeichnet, daß man ein Verhältnis Halogen : N oder P von größer als 1 einhält.

9. Verfahren nach Anspruch 1 bis 8, dadurch gekennzeichnet, daß man die Umsetzung bei einer Temperatur von 160 bis 220 °C und Reaktionsdrücken von 10 bis 80 bar durchführt.

10. Verfahren nach Anspruch 1 bis 9, dadurch gekennzeichnet, daß man die Umsetzung in Gegenwart von Kohlenmonoxid durchführt.

11. Verfahren nach Anspruch 1 bis 10, dadurch gekennzeichnet, daß man bei der Umsetzung Carbonsäureester und Ameisensäure in äquimolaren Mengen einsetzt.

12. Verfahren nach Anspruch 1 bis 11, dadurch gekennzeichnet, daß man als Carbonsäureester einen Methyl- oder Benzylester einsetzt.

13. Katalysatorsystem zur Herstellung von Carbonsäuren durch Umsetzung von Carbonsäureestern mit Ameisensäure nach den Ansprüchen 1 bis 12, dadurch gekennzeichnet, daß das Katalysatorsystem aus Rhodium bzw. einer Rhodiumverbindung, einem Halogen oder einer Halogenverbindung als Promotor, aus einem Salz oder Komplex eines Elementes der VI. Nebengruppe und/oder aus Liganden aus der Gruppe der organischen Stickstoff- oder Phosphorverbindungen besteht, wobei das Atomverhältnis des Rhodiums : Halogen 1 : 1 000 bis 1 : 1, das des Rhodiums : Metall der VI. Nebengruppe 1 : 100 bis 10 : 1 und das des Rh : N oder P 1 : 100 bis 1 : 1 beträgt.